# EUROPEAN PATENT APPLICATION

(11) **EP 3 072 939 A1**
(43) Date of publication of application: **28.09.2016**
(21) Application number: 15177403.1
(22) Date of filing: 17.07.2015
(51) Int. Cl.: C09K 11/02, C09K 11/56, C09K 11/88, G01N 33/58

(54) **NANOPLATELETS AND HIGH TEMPERATURE PROCESS FOR MANUFACTURE THEREOF**

(30) Priority: 27.03.2015 US 201562139150 P
(71) Applicant: Nexdot, 75005 Paris (FR)
(72) Inventor: HEUCLIN, Hadrien, 75005 Paris (FR); NADAL, Brice, 75005 Paris (FR)
(74) Representative: Icosa

(57) **Abstract**

The present invention relates to a population of semiconductor nanoplatelets, each member of the population comprising a nanoplatelet core including a first semiconductor material and a shell including a second semiconductor material on the surface of the nanoplatelet core, wherein after ligand exchange reaction the population exhibits a quantum yield decrease of less than 50%.The present invention also relates to a high temperature growing process for manufacturing said nanoplatelets.

## Description

### FIELD OF INVENTION

The present invention relates to the field of nanoparticles and especially semiconductor nanoplatelets. In particular, the present invention relates to nanoplatelets exhibiting improved features, to a high temperature process for manufacturing nanoplatelets and to nanoplatelets obtained therefrom.

### BACKGROUND OF INVENTION

It is known that semiconductor nanoparticles, such as quantum dots or nanoplatelets, exhibits desirable properties: narrow fluorescence full width at half maximum, large absorption cross-section, tunable emission wavelength, etc.

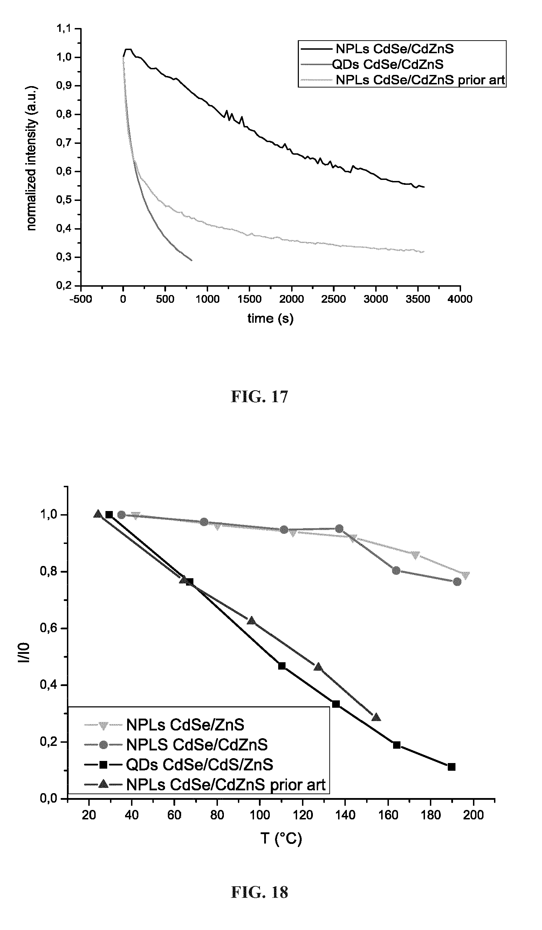

However, existing quantum dots and nanoplatelets exhibit stability in time and in temperature which is not yet sufficient for large scale industrial applications (see figs. 17-19).

It is therefore an object of the present invention to provide nanoplatelets with enhanced fluorescence properties, especially enhanced fluorescence quantum yield, enhanced resistance to photobleaching, enhanced resistance to light flux and enhanced fluorescence stability over temperature variations.

### SUMMARY

Thus the present invention relates to a population of semiconductor nanoplatelets, each member of the population comprising a nanoplatelet core including a first semiconductor material and a shell including a second semiconductor material on the surface of the nanoplatelet core, wherein after ligand exchange reaction the population exhibits a quantum yield decrease of less than 50%.

According to one embodiment, the ligand is selected from a carboxylic acid, a thiol, an amine, a phosphine, a phosphine oxide an amide, an ester, a pyridine, an imidazole and/or an alcohol.

According to one embodiment, the nanoplatelets population exhibits fluorescence quantum efficiency decrease of less than 50% after one hour under light illumination.

According to one embodiment, the population exhibits fluorescence quantum efficiency at 100 °C or above that is at least 80% of the fluorescence quantum efficiency of the population at 20°C. According to one embodiment, the temperature is in a range from 100 °C to 250°C.

The present invention also relates to a process of growth of a ME shell on initial colloidal nanoplatelets; said process comprising the steps of injecting the initial colloidal nanoplatelets in a solvent at a temperature ranging from 200°C to 460°C and subsequently a precursor of E or M, wherein said precursor of E or M is injected slowly in order to control the shell growth rate; and wherein the precursor of respectively M or E is injected either in the solvent before injection of the initial colloidal nanoplatelets or in the mixture simultaneously with the precursor of respectively E or M, wherein:
M is selected from group Ib, IIa, IIb, IIIa, IIIb, IVa, IVb, Va, Vb, VIb, VIIb, VIII or mixtures thereof;
E is selected from group Va, VIa, VIIa or mixtures thereof; and
x and y are independently a decimal number from 0 to 5.

According to one embodiment, the process comprises successively the steps of:
- heating a solvent at a temperature ranging from 200°C to 460°C;
- injecting in the solvent the initial colloidal nanoplatelets;
- injecting slowly in the mixture the precursor of E and the precursor of M;
- recovering the core/shell nanoplatelets comprising a ME shell on the initial nanoplatelets in the form of nanoplatelets.

According to one embodiment, the process comprises successively the steps of:
- heating a solvent at a temperature ranging from 200°C to 460°C;
- injecting a precursor of M in the solvent;
- injecting in the mixture the initial colloidal nanoplatelets;
- injecting slowly in the mixture the precursor of E;
- recovering the core/shell nanoplatelets comprising a ME shell on the initial nanoplatelets in the form of nanoplatelets.

According to one embodiment, the process comprises successively the steps of:
- heating a solvent at a temperature ranging from 200°C to 460°C;
- injecting a precursor of E in the solvent;
- injecting in the mixture the initial colloidal nanoplatelets;
- injecting slowly in the mixture the precursor of M;
- recovering the core/shell nanoplatelets comprising a ME shell on the initial nanoplatelets in the form of nanoplatelets.

According to one embodiment, the process comprises successively the steps of:
- providing a solvent and a precursor of M;
- heating the mixture of the solvent and the precursor of M at a temperature ranging from 200°C to 460°C;
- injecting in the mixture the initial colloidal nanoplatelets;
- injecting slowly in the mixture the precursor of E;
- recovering in the form of nanoplatelets the core/shell nanoplatelets comprising a ME shell on the initial nanoplatelets.

According to one embodiment, the process comprises successively the steps of:
- providing a solvent and a precursor of E;
- heating the mixture of the solvent and the precursor of E at a temperature ranging from 200°C to 460°C;
- injecting in the mixture the initial colloidal nanoplatelets;
- injecting slowly in the mixture the precursor of M;
- recovering in the form of nanoplatelets the core/shell nanoplatelets comprising a ME shell on the initial nanoplatelets.

According to one embodiment, a fraction of the precursor's mixture is mixed with the initial colloidal nanoplatelets before injection in the solvent.

According to one embodiment, the process further comprises the step of maintaining the mixture at a temperature ranging from 200°C to 460°C during a predetermined duration ranging from 5 to 180 minutes after injection of the second precursor.

According to one embodiment, the slow injection of the at least one of the precursor of E or M is performed at a rate ranging from 0.1 to 30 mole/h/mole of M present in the initial nanoplatelets.

According to one embodiment, the initial colloidal nanoplatelets are injected over a period of less than 1 minute.

### DEFINITIONS

In the present invention, the following terms have the following meanings:
- As used herein the singular forms **"a", "an",** and **"the"** include plural reference unless the context clearly dictates otherwise.
- The term **"about"** is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent.
- **"Continuously emissive nanoplatelets"** over a predetermined period refers to nanoplatelets which exhibit, under excitation, fluorescence (or photoluminescence) intensity above a threshold over the predetermined period. The integration time is set to allow sufficient excitation events of the nanoplatelets and is superior or equal to 1 ms. According to the present invention, during a measurement (see examples), said threshold may be set at three times the noise.
- **"Fluorescence lifetime"** refers to the average time an excited fluorophore remains in the excited state before it emits a photon and decays to the ground state.
- **"Fluorescence quantum efficiency or quantum yield"** refers to the ratio between the number of photons emitted by fluorescence divided by the number of absorbed photons.
- **"Monolayer"** refers to a film or a continuous layer being of one atom thick.
- **"Nanoparticle or nanocrystal"** refers to a particle of any shape having at least one dimension in the 0.1 to 100 nanometers range.
- **"Nanoplatelet", "nanosheet", "nanoplate"** or **"2D-nanoparticle"** refers interchangeably to a nanoparticle having one dimension smaller than the two others; said dimension ranging from 0.1 to 100 nanometers. In the sense of the present invention, the smallest dimension (hereafter referred to as the thickness) is smaller than the other two dimensions (hereafter referred to as the length and the width) by a factor of at least 1.5, 2, 2.5, 3, 3.5, 4.5 or 5.
- **"On-time fraction"** refers to the fraction of time during continuous excitation in which a particle is exhibiting fluorescence emission, referred to as being "on".
- **"Shell"** refers to a film or a layer of at least one atom thick covering the initial nanoplatelet on each faces (i.e. on the entire surface except, if the growth process is performed on a substrate, on the surface in contact with said substrate).
- **"Scattering element"** refers to an optical element that diffuses, spreads out or scatters light. Illustrative scattering element includes light scattering film, surface structuration, particulate-filled composite and combinations thereof.

### DETAILED DESCRIPTION

This invention relates to a nanoplatelet comprising an initial nanoplatelet core and a shell.

According to a first embodiment, the initial nanoplatelet is an inorganic, colloidal, semiconductor and/or crystalline nanoplatelet.

According to one embodiment, the initial nanoplatelet has a thickness ranging from 0.3 nm to less than 500 nm, from 5 nm to less than 250 nm, from 0.3 nm to less than 100 nm, from 0.3 nm to less than 50 nm, from 0.3 nm to less than 25 nm, from 0.3 nm to less than 20 nm, from 0.3 nm to less than 15 nm, from 0.3 nm to less than 10 nm, or from 0.3 nm to less than 5 nm.

According to one embodiment, at least one of the lateral dimensions of the initial nanoplatelet is ranging from 2 nm to 1m, from 2 nm to 100 mm, from 2 nm to 10 mm, from 2 nm to 1 mm, from 2 nm to 100 µm, from 2 nm to 10 µm, from 2 nm to 1 µm, from 2 nm to 100 nm, or from 2 nm to 10 nm.

According to one embodiment, the material composing the initial nanoplatelet comprises a material MxEy, wherein:
M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is selected from O, S, Se, Te, N, P, As, F, Cl, Br, I, or a mixture thereof; and
x and y are independently a decimal number from 0 to 5.

According to an embodiment, the material MxEy comprises cationic element M and anionic element E in stoichiometric ratio, said stoichiometric ratio being characterized by values of x and y corresponding to absolute values of mean oxidation number of elements E and M respectively.

According to one embodiment, the faces substantially normal to the axis of the smallest dimension of the initial nanoplatelet consist either of M or E.

According to one embodiment, the smallest dimension of the initial nanoplatelet comprises an alternate of atomic layers of M and E.

According to one embodiment, the number of atomic layers of M in the initial nanoplatelet is equal to one plus the number of atomic layer of E.

According to an embodiment, the material composing the initial nanoplatelet comprises a material MxNyEz, wherein:
- M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- N is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- E is selected from O, S, Se, Te, N, P, As, F, Cl, Br, I or a mixture thereof; and x, y and z are independently a decimal number from 0 to 5, at the condition that when x is 0, y and z are not 0, when y is 0, x and z are not 0 and when z is 0, x and y are not 0.

According to a preferred embodiment, the material composing the initial nanoplatelet comprises a material MxEy wherein:
M is selected from group Ib, IIa, IIb, IIIa, IIIb, IVa, IVb, Va, Vb, VIb, VIIb, VIII or mixtures thereof;
E is selected from group Va, VIa, VIIa or mixtures thereof; and
x and y are independently a decimal number from 0 to 5.

According to one embodiment, the material composing the initial nanoplatelet comprises a semi-conductor from group IIb-VIa, group IVa-VIa, group Ib-IIIa-VIa, group IIb-IVa-Va, group Ib-VIa, group VIII-VIa, group IIb-Va, group IIIa-VIa, group IVb-VIa, group IIa-VIa, group IIIa-Va, group IIIa-VIa, group VIb-VIa, or group Va-VIa.

According to one embodiment, the material composing the initial nanoplatelet comprises at least one semiconductor chosen among CdS, CdSe, CdTe, CdO, Cd₃P₂, Cd₃As₂, ZnS, ZnSe, ZnO, ZnTe, Zn₃P₂, Zn3As2, HgS, HgSe, HgTe, HgO, GeS, GeSe, GeTe, SnS, SnS₂, SnSe₂, SnSe, SnTe, PbS, PbSe, PbTe, GeS₂, GeSe₂, CuInS₂, CuInSe₂, CuS, Cu₂S, Ag₂S, Ag₂Se, Ag₂Te AgInS₂, AgInSe₂, FeS, FeS₂, FeO, Fe₂O₃, Fe₃O₄, Al₂O₃, TiO₂, MgO, MgS, MgSe, MgTe, AlN, AlP, AlAs, AlSb, GaN, GaP, GaAs, GaSb, InN, InP, InAs, InSb, In₂S₃,TlN, TlP, TlAs, TlSb, Bi₂S₃, Bi₂Se₃, Bi₂Te₃, MoS₂, WS₂, VO₂ or a mixture thereof.

According to a preferred embodiment, the initial nanoplatelet is selected from the group consisting of CdS, CdSe, CdSSe, CdTe, ZnS, ZnSe, ZnTe, PbS, PbSe, PbTe, CuInS₂, CuInSe₂, AgInS₂, AgInSe₂, CuS, Cu₂S, Ag₂S, Ag₂Se, Ag₂Te, FeS, FeS₂, PdS, Pd₄S, WS₂ or a mixture thereof.

According to one embodiment, the initial nanoplatelet comprises an alloy of the aforementioned materials.

According to one embodiment, the initial nanoplatelet comprises an additional element in minor quantities. The term "minor quantities" refers herein to quantities ranging from 0.0001 % to 10% molar, preferably from 0.001 % to 10% molar.

According to one embodiment, the initial nanoplatelet comprises a transition metal or a lanthanide in minor quantities. The term "minor quantities" refers herein to quantities ranging from 0.0001 % to 10% molar, preferably from 0.001 % to 10% molar.

According to one embodiment, the initial nanoplatelet comprises in minor quantities an element inducing an excess or a defect of electrons compared to the sole nanoplatelet. The term "minor quantities" refers herein to quantities ranging from 0.0001% to 10% molar, preferably from 0.001% to 10% molar.

According to one embodiment, the initial nanoplatelet comprises in minor quantities an element inducing a modification of the optical properties compared to the sole nanoplatelet. The term "minor quantities" refers herein to quantities ranging from 0.0001 % to 10% molar, preferably from 0.001 % to 10% molar.

According to one embodiment, the initial nanoplatelet consists of a core/shell nanoplatelet such as a core/shell nanoplatelet known by one skilled in the art or a core/shell nanoplatelet according to the present invention. According to one embodiment, the "core" nanoplatelets can have an overcoating or shell on the surface of its core.

According to a first embodiment, the final nanoplatelet is an inorganic, colloidal, semiconductor and/or crystalline nanoplatelet.

According to one embodiment, the final nanoplatelet has a thickness ranging from 0.5 nm to 10 mm, from 0.5 nm to 1 mm, from 0.5 nm to 100 µm, from 0.5 nm to 10 µm, from 0.5 nm to 1 µm, from 0.5 nm to 500 nm, from 0.5 nm to 250 nm, from 0.5 nm to 100 nm, from 0.5 nm to 50 nm, from 0.5 nm to 25 nm, from 0.5 nm to 20 nm, from 0.5 nm to 15 nm, from 0.5 nm to 10 nm or from 0.5 nm to 5 nm.

According to one embodiment, at least one of the lateral dimensions of the final nanoplatelet is ranging from 2 nm to 1m, from 2 nm to 100 mm, from 2 nm to 10 mm, from 2 nm to 1 mm, from 2 nm to 100 µm, from 2 nm to 10 µm, from 2 nm to 1 µm, from 2 nm to 100 nm, or from 2 nm to 10 nm.

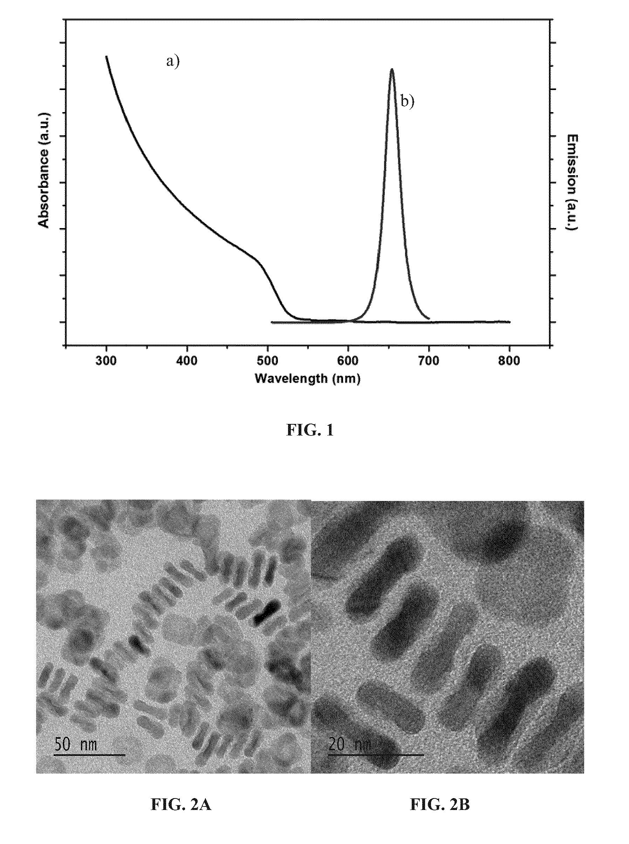

According to one embodiment, the thickness of the shell is ranging from 0.2 nm to 10 mm, from 0.2 nm to 1 mm, from 0.2 nm to 100 µm, from 0.2 nm to 10 µm, from 0.2 nm to 1 µm, from 0.2 nm to 500 nm, from 0.2 nm to 250 nm, from 0.2 nm to 100 nm, from 0.2 nm to 50 nm, from 0.2 nm to 25 nm, from 0.2 nm to 20 nm, from 0.2 nm to 15 nm, from 0.2 nm to 10 nm or from 0.2 nm to 5 nm (Figures 2; 3; 4; 11; 13; 14 and 16).

According to one embodiment, the material composing the shell comprises a material MxEy, wherein:
M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is selected from O, S, Se, Te, N, P, As, F, Cl, Br, I, or a mixture thereof,
and x and y are independently a decimal number from 0 to 5.

According to an embodiment, the material MxEy comprises cationic element M and anionic element E in stoichiometric ratio, said stoichiometric ratio being characterized by values of x and y corresponding to absolute values of mean oxidation number of elements E and M respectively.

According to one embodiment, the faces substantially normal to the axis of the smallest dimension of the shell consist either of M or E.

According to one embodiment, the smallest dimension of the shell comprises either an alternate of atomic layers of M and E.

According to one embodiment, the number of atomic layers of M in the shell is equal to one plus the number of atomic layer of E.

According to an embodiment, the material composing the shell comprises a material MxNyEz, wherein:
- M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- N is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- E is selected from O, S, Se, Te, N, P, As, F, Cl, Br, I, or a mixture thereof; and x, y and z are independently a decimal number from 0 to 5, at the condition that when x is 0, y and z are not 0, when y is 0, x and z are not 0 and when z is 0, x and y are not 0.

According to a preferred embodiment, the material composing the shell comprises a material MxEy wherein:
M is selected from group Ib, IIa, IIb, IIIa, IIIb, IVa, IVb, Vb, VIb, VIIb, VIII or mixtures thereof;
E is selected from group Va, VIa, VIIa or mixtures thereof; and
x and y are independently a decimal number from 0 to 5.

According to one embodiment, the material composing the shell comprises a semi-conductor from group IIb-VIa, group IVa-VIa, group Ib-IIIa-VIa, group IIb-IVa-Va, group Ib-VIa, group VIII-VIa, group IIb-Va, group IIIa-VIa, group IVb-VIa, group IIa-VIa, group IIIa-Va, group IIIa-VIa, group VIb-VIa, or group Va-VIa.

According to one embodiment, the material composing the shell comprises at least one semiconductor chosen among CdS, CdSe, CdTe, CdO, Cd₃P₂, Cd₃As₂, ZnS, ZnSe, ZnO, ZnTe, Zn₃P₂, Zn3As2, HgS, HgSe, HgTe, HgO, GeS, GeSe, GeTe, SnS, SnS₂, SnSe₂, SnSe, SnTe, PbS, PbSe, PbTe, GeS₂, GeSe₂, CuInS₂, CuInSe₂, CuS, Cu₂S, Ag₂S, Ag₂Se, Ag₂Te AgInS₂, AgInSe₂, FeS, FeS₂, FeO, Fe₂O₃, Fe₃O₄, Al₂O₃, TiO₂, MgO, MgS, MgSe, MgTe, AlN, AlP, AlAs, AlSb, GaN, GaP, GaAs, GaSb, InN, InP, InAs, InSb, In₂S₃,TlN, TlP, TlAs, TlSb, Bi₂S₃, Bi₂Se₃, Bi₂Te₃, MoS₂, WS₂, VO₂ or a mixture thereof.

According to one embodiment, the shell comprises an alloy of the aforementioned materials.

According to a preferred embodiment, the shell is selected from the group consisting of CdS, CdSSe, CdSe, CdTe, ZnS, ZnSe, ZnTe, PbS, PbSe, PbTe, CuInS₂, CuInSe₂, AgInS₂, AgInSe₂, CuS, Cu₂S, Ag₂S, Ag₂Se, Ag₂Te, FeS, FeS₂, PdS, Pd₄S, WS₂ or a mixture thereof.

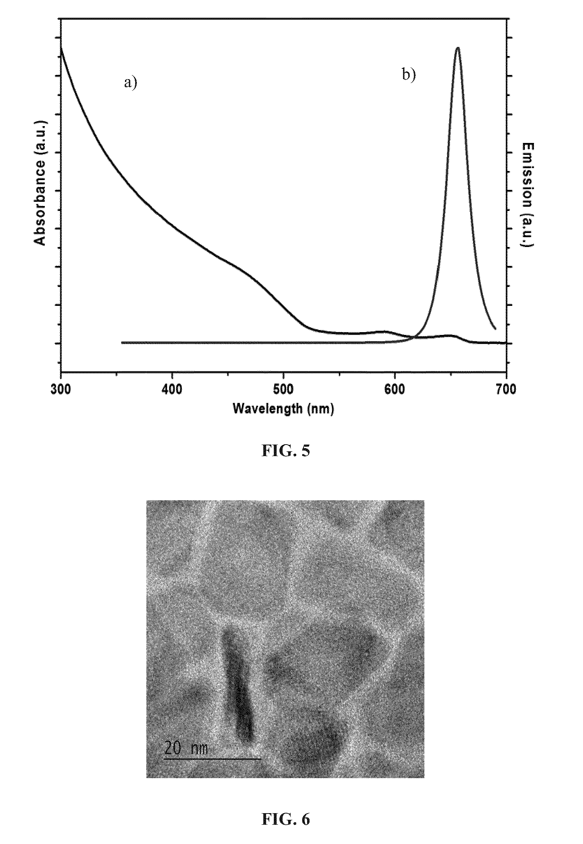

According to a preferred embodiment, the final core/shell nanoplatelet is selected from the group consisting of CdSe/CdS (Figure 1); CdSe/CdZnS (Figures 5; 6; 7 and 8); CdSe/ZnS; CdSeTe/CdS; CdSeTe/CdZnS; CdSeTe/ZnS; CdSSe/CdS; CdSSe/CdZnS; CdSSe/ZnS.

According to a preferred embodiment, the final core/shell nanoplatelet is selected from the group consisting of CdSe/CdS/ZnS; CdSe/CdZnS/ZnS; CdSeTe/CdS/ZnS; CdSeTe/CdZnS/ZnS; CdSeTe/ZnS; CdSSe/CdS/ZnS; CdSSe/CdZnS/ZnS; CdSSe/ZnS.

According to one embodiment, the final nanoplatelet is homostructured, i.e. the initial nanoplatelet and the shell are composed of the same material.

In one embodiment, the final nanoplatelet is heterostructured, i.e. the initial nanoplatelet and the shell are composed of at least two different materials.

According to one embodiment, the final nanoplatelet comprises the initial nanoplatelet and a sheet comprising at least one layer covering all of the initial nanoplatelet. Said layer being composed of the same material as the initial nanoplatelet or a different material than the initial nanoplatelet.

According to one embodiment, the final nanoplatelet comprises the initial nanoplatelet and a shell comprising 1, 2, 3, 4, 5, 6, 7, 8, 9, 10, 15, 20, 25, 50 or more monolayers covering all of the initial nanoplatelet. Said layers being of same composition as the initial nanoplatelet or being of different composition than the initial nanoplatelet or being of different composition one to another.

According to one embodiment, the final nanoplatelet comprises the initial nanoplatelet and a shell comprising at least 5, 6, 7, 8, 9, 10, 15, 20, 25, 50 or more monolayers covering all of the initial nanoplatelet. Said layers being of same composition as the initial nanoplatelet or being of different composition than the initial nanoplatelet or being of different composition one to another.

According to one embodiment, the faces substantially normal to the axis of the smallest dimension of the final nanoplatelet consist either of M or E.

According to one embodiment, the smallest dimension of the final nanoplatelet comprises either an alternate of atomic layers of M and E.

According to one embodiment, the number of atomic layers of M in the final nanoplatelet is equal to one plus the number of atomic layer of E.

According to one embodiment, the shell is homogeneous thereby producing a final nanoplatelet.

According to one embodiment, the shell comprises a substantially identical thickness on each facet on the initial nanoplatelet.

The present invention relates to a process of growth of a shell on initial colloidal nanoplatelets.

According to one embodiment, the initial nanoplatelet is obtained by any method known from one skilled in the art.

According to one embodiment, the process of growth of a shell comprises the growth of an homogeneous shell on each facet of the initial colloidal nanoplatelet.

According to one embodiment, the process of growth of a ME shell on initial colloidal nanoplatelets comprises the steps of injecting the initial colloidal nanoplatelets in a solvent at a temperature ranging from 200°C to 460°C and subsequently a precursor of E or M, wherein said precursor of E or M is injected slowly in order to control the shell growth rate; and wherein the precursor of respectively M or E is injected either in the solvent before injection of the initial colloidal nanoplatelets or in the mixture simultaneously with the precursor of respectively E or M.

According to one embodiment, the initial colloidal nanoplatelets are mixed with a fraction of the precursor's mixture before injection in the solvent.

According to one embodiment, the process of growth of a MxEy shell on initial colloidal nanoplatelets comprises the steps of injecting the initial colloidal nanoplatelets in a solvent at a temperature ranging from 200°C to 460°C and subsequently a precursor of E or M, wherein said precursor of E or M is injected slowly in order to control the shell growth rate; and wherein the precursor of respectively M or E is injected either in the solvent before injection of the initial colloidal nanoplatelets or in the mixture simultaneously with the precursor of respectively E or M; wherein x and y are independently a decimal number from 0 to 5.

According to one embodiment, the process of growth of a ME shell on initial colloidal nanoplatelets comprises the following steps:
- heating a solvent at a temperature ranging from 200°C to 460°C;
- injecting in the solvent the initial colloidal nanoplatelets;
- injecting slowly in the mixture the precursor of E and the precursor of M;
- recovering the core/shell structure in the form of nanoplatelets.

According to another embodiment, the process of growth of a ME shell on initial colloidal nanoplatelets comprises the following steps:
- heating a solvent at a temperature ranging from 200°C to 460°C;
- injecting a precursor of M in the solvent;
- injecting in the mixture the initial colloidal nanoplatelets;
- injecting slowly in the mixture the precursor of E;
- recovering the core/shell structure in the form of nanoplatelets.

According to another embodiment, the process of growth of a ME shell on initial colloidal nanoplatelets comprises the following steps:
- heating a solvent at a temperature ranging from 200°C to 460°C;
- injecting a precursor of E in the solvent;
- injecting in the mixture the initial colloidal nanoplatelets;
- injecting slowly in the mixture the precursor of M;
- recovering the core/shell structure in the form of nanoplatelets.

According to another embodiment, the process of growth of a ME shell on initial colloidal nanoplatelets comprises the following steps:
- heating a solvent at a temperature ranging from 200°C to 460°C;
- injecting in the solvent the initial colloidal nanoplatelets, optionally mixed with a fraction of the precursors mixture;
- injecting slowly in the mixture the precursor of E and the precursor of M;
- recovering the core/shell structure in the form of nanoplatelets.

Herein the term "fraction of the precursors mixture" refers to a part of the total amount of precursors used in the reaction, i.e. from 0.001% to 50 %, preferably from 0.001% to 25%, more preferably from 0.01% to 10% of the total amount of the injected precursors mixture.

According to another embodiment, the process of growth of core/shell nanoplatelets comprising a ME shell on initial colloidal nanoplatelets comprises the following steps:
- providing a solvent and a precursor of M;
- heating the mixture of the solvent and the precursor of M at a temperature ranging from 200°C to 460°C;
- injecting in the mixture the initial colloidal nanoplatelets;
- injecting slowly in the mixture the precursor of E;
- recovering in the form of nanoplatelets the core/shell structure.

According to another embodiment, the process of growth of core/shell nanoplatelets comprising a ME shell on initial colloidal nanoplatelets comprises the following steps:
- providing a solvent and a precursor of E;
- heating the mixture of the solvent and the precursor of E at a temperature ranging from 200°C to 460°C;
- injecting in the mixture the initial colloidal nanoplatelets;
- injecting slowly in the mixture the precursor of M;
- recovering in the form of nanoplatelets the core/shell structure.

According to one embodiment, the initial colloidal nanoplatelets have a core/shell structure.

According to one embodiment, the process of growth of core/shell nanoplatelets comprising a ME shell on initial colloidal nanoplatelets further comprises the step of maintaining the mixture at a temperature ranging from 200°C to 460°C during a predetermined duration ranging from 1 to 180 minutes after the end of the injection of the second precursor.

According to one embodiment, the temperature of the annealing ranges from 200°C and 460°C, from 275°C to 365°C, from 300°C to 350°C or about 300°C.

According to one embodiment, the duration of the annealing ranges from 1 to 180 minutes, from 30 to 120 minutes, from 60 to 120 minutes or about 90 minutes.

According to one embodiment, the initial colloidal nanoplatelets are injected over a period of less than 10 minutes, less than 5 minutes, less than 1 minute, less than 30 seconds, less than 10 seconds, less than 5 seconds or less than 1 second. According to one embodiment, the initial colloidal nanoplatelets are injected at once.

According to one embodiment, the initial colloidal nanoplatelets are injected at a rate ranging from 1 mL/s to 1 L/s, from 1 mL/s to 100 mL/s, from 1 mL/s to 10 mL/s, from 2 to 8 mL/s or about 5 mL/s.

According to one embodiment, the injection of the precursor of E or the precursor of M of the shell is performed at a rate ranging from 0.1 to 30 mole/h/mole of M present in the initial nanoplatelet, preferably from 0.2 to 20 mole/h/mole of M present in the initial nanoplatelet, more preferably from 1 to 21 mole/h/mole of M present in the initial nanoplatelets.

According to one embodiment, the precursor of E or the precursor of M is injected slowly i.e. over a period ranging from 1 minutes to 2 hours, from 1 minute to 1 hour, from 5 to 30 minutes or from 10 to 20 minutes for each monolayer.

According to one embodiment, the precursor of E is injected slowly, i.e. at a rate ranging from 0.1 mL/h to 10 L/h, from 0.5 mL/h to 5 L/h or from 1 mL/h to 1 L/h.

According to one embodiment, the precursor of M is injected slowly, i.e. at a rate ranging from 0.1 mL/h to 10 L/h, from 0.5 mL/h to 5 L/h or from 1 mL/h to 1 L/h.

According to one embodiment, the precursor of E and the precursor of M are injected slowly in order to control the shell growth rate.

According to one embodiment wherein the precursor of M or the precursor of E is injected prior to the initial colloidal nanoplatelets, said precursor of M or said precursor of E is injected over a period of less than 30 seconds, less than 10 seconds, less than 5 seconds, less than 1 second. According to another embodiment wherein the precursor of M or the precursor of E is injected prior to the initial colloidal nanoplatelets, said precursor of M or said precursor of E is injected slowly, i.e. at a rate ranging from 0.1 mL/h to 10 L/h, from 0.5 mL/h to 5 L/h or from 1 mL/h to 1 L/h.

According to one embodiment, the precursor of M or the precursor of E injected prior to the initial colloidal nanoplatelets is injected faster than the precursor of M or the precursor of E injected after the initial colloidal nanoplatelets.

According to one embodiment, the injection's rate of at least one of the precursor of E and/or the precursor of M is chosen such that the growth rate of the shell is ranging from 1 nm per second to 0.1 nm per hour.

According to one embodiment, the growth process is performed at temperature ranging from 200°C to 460°C, from 275°C to 365°C, from 300°C to 350°C or about 300°C. According to one embodiment, the reaction is performed under an inert atmosphere, preferably nitrogen or argon atmosphere.

According to one embodiment, the precursor of E is capable of reacting with the precursor of M to form a material with the general formula ME.

According to one embodiment, the precursor of the shell to be deposited is a precursor of a material MxEy, wherein:
M is Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is O, S, Se, Te, N, P, As, F, Cl, Br, I, or a mixture thereof,
and x and y are independently a decimal number from 0 to 5.

According to an embodiment, the precursor of the shell to be deposited is a material MxEy comprising cationic element M and anionic element E in stoichiometric ratio, said stoichiometric ratio being characterized by values of x and y corresponding to absolute values of mean oxidation number of elements E and M respectively.

According to a preferred embodiment, the precusrsor of the shell to be deposited is a precursor of a material MxEy wherein:
M is selected from group Ib, IIa, IIb, IIIa, IIIb, IVa, IVb, Vb, VIb, VIIb, VIII or mixtures thereof;
E is selected from group Va, VIa, VIIa or mixtures thereof; and
x and y are independently a decimal number from 0 to 5.

According to one embodiment, the precursor of the shell to be deposited is a precursor of a compound of group IIb-VIa, group IVa-VIa, group Ib-IIIa-VIa, group IIb-IVa-Va, group Ib-VIa, group VIII-VIa, group IIb-Va, group IIIa-VIa, group IVb-VIa, group IIa-VIa, group IIIa-Va, group IIIa-VIa, group VIb-VIa, or group Va-VIa.

According to one embodiment, the precursor of the shell to be deposited is a precursor of a material chosen among CdS, CdSe, CdTe, CdO, Cd₃P₂, Cd₃As₂, ZnS, ZnSe, ZnO, ZnTe, Zn₃P₂, Zn3As2, HgS, HgSe, HgTe, HgO, GeS, GeSe, GeTe, SnS, SnS₂, SnSe₂, SnSe, SnTe, PbS, PbSe, PbTe, GeS₂, GeSe₂, CuInS₂, CuInSe₂, CuS, Cu₂S, Ag₂S, Ag₂Se, Ag₂Te AgInS₂, AgInSe₂, FeS, FeS₂, FeO, Fe₂O₃, Fe₃O₄, Al₂O₃, TiO₂, MgO, MgS, MgSe, MgTe, AlN, AlP, AlAs, AlSb, GaN, GaP, GaAs, GaSb, InN, InP, InAs, InSb, In₂S₃,TlN, TlP, TlAs, TlSb, Bi₂S₃, Bi₂Se₃, Bi₂Te₃, MoS₂, WS₂, VO₂ or a mixture thereof.

According to a preferred embodiment, the precursor of the shell to be deposited is a precursor of a material selected from the group consisting of CdS, CdSSe, CdSe, CdTe, ZnO, ZnS, ZnSe, ZnTe, PbS, PbSe, PbTe, CuInS₂, CuInSe₂, AgInS₂, AgInSe₂, CuS, Cu₂S, Ag₂S, Ag₂Se, Ag₂Te, FeS, FeS₂, PdS, Pd₄S, WS₂ or a mixture thereof.

According to one embodiment, if E is a chalcogenide, the precursor of E is a compound containing the chalcogenide at the -2 oxidation state. According to one embodiment, if E is a chalcogenide, the precursor of E is formed in situ by reaction of a reducing agent with a compound containing E at the 0 oxidation state or at a strictly positive oxidation state.

According to one embodiment, if E is sulfur, the precursor of E is a thiol. According to one embodiment, if E is sulfur, the precursor of E is dodecanethiol or octanethiol. According to one embodiment, if E is sulfur, the precursor of E is a salt containing S²⁻sulfide ions. According to one embodiment, if E is sulfur, the precursor of E comprises bis(trimethylsilyl) sulfide (TMS₂S) or hydrogen sulfide (H₂S) or sodium hydrogen sulfide (NaSH) or sodium sulfide (Na₂S) or ammonium sulfide (S(NH₄)₂) or thiourea or thioacetamide. According to one embodiment, if E is sulfur, the precursor of E is sulfur dissolved in a suitable solvent. According to one embodiment, if E is sulfur, the precursor of E is sulfur dissolved in 1-octadecene. According to one embodiment, if E is sulfur, the precursor of E is sulfur dissolved in a phosphine. According to one embodiment, if E is sulfur, the precursor of E is sulfur dissolved in trioctylphosphine or tributylphosphine. According to one embodiment, if E is sulfur, the precursor of E is sulfur dissolved in an amine. According to one embodiment, if E is sulfur, the precursor of E is sulfur dissolved in oleylamine. According to one embodiment, if E is sulfur, the precursor of E is sulfur powder dispersed in a solvent. According to one embodiment, if E is sulfur, the precursor of E is sulfur powder dispersed in 1-octadecene.

According to one embodiment, if E is selenium, the precursor of E comprises a salt containing Se²⁻ selenide ions. According to one embodiment, the precursor of E comprises bis(trimethylsilyl) selenide (TMS₂Se) or hydrogen selenide (H₂Se) or sodium selenide (Na₂Se) or sodium hydrogen selenide (NaSeH) or sodium selenosulfate (Na₂SeSO₃) or selenourea. According to one embodiment, if E is selenium, the precursor of E is a selenol. According to one embodiment, if E is selenium, the precursor of E is a diselenide, such as Diphenyl diselenide. According to one embodiment, if E is selenium, the precursor of E is selenium dissolved in a suitable solvent. According to one embodiment, if E is selenium, the precursor of E is selenium dissolved in 1-octadecene. According to one embodiment, if E is selenium, the precursor of E is selenium dissolved in a phosphine. According to one embodiment, if E is selenium, the precursor of E is selenium dissolved in trioctylphosphine or tributylphosphine. According to one embodiment, if E is selenium, the precursor of E is selenium dissolved in an amine. According to one embodiment, if E is selenium, the precursor of E is selenium dissolved in an amine and thiol mixture. According to one embodiment, if E is selenium, the precursor of E is selenium powder dispersed in a solvent. According to one embodiment, if E is selenium, the precursor of E is selenium powder dispersed in 1-octadecene.

According to one embodiment, if E is tellurium, the precursor of E is as salt containing Te²⁻ telluride ions. According to one embodiment, if E is tellurium, the precursor of E comprises bis(trimethylsilyl) telluride (TMS₂Te) or hydrogen telluride (H₂Te) or sodium telluride (Na₂Te) or sodium hydrogen telluride (NaTeH) or sodium tellurosulfate (Na₂TeSO₃) or tellurourea. According to one embodiment, if E is tellurium, the precursor of E is tellurium dissolved in a suitable solvent. According to one embodiment, if E is tellurium, the precursor of E is tellurium dissolved a phosphine. According to one embodiment, if E is tellurium, the precursor of E is tellurium dissolved in trioctylphosphine or tributylphosphine.

According to one embodiment, if E is oxygen, the precursor of E is the hydroxide ion (HO⁻). According to one embodiment, if E is oxygen the precursor of E is a solution of sodium hydroxide (NaOH) or of potassium hydroxide (KOH) or of tetramethylammonium hydroxide (TMAOH). According to one embodiment, if E is oxygen, the precursor of E is generated in-situ by condensation between an amine and a carboxylic acid. According to one embodiment, if E is oxygen, the precursor of E is generated in-situ by condensation of two carboxylic acids.

According to one embodiment, if E is phosphorus, the precursor of E comprises phosphorus at the -3 oxidation state. According to one embodiment, the precursor of E comprises tris(trimethylsilyl) phosphine (TMS₃P) or phosphine (PH₃) or white phosphorus (P₄) or phosphorus trichloride (PCl₃). According to one embodiment, the precursor of E comprises a tris(dialkylamino)phosphine for example tris(dimethylamino)phosphine ((Me₂N)₃P) or tris(diethylamino)phosphine ((Et₂N)₃P). According to one embodiment, the precursor of E comprises a trialkylphosphine for example trioctylphosphine or tributylphosphine or triphenylphosphine.

According to one embodiment, if M is a metal, the precursor of M is a compound containing the metal at positive or 0 oxidation state. According to one embodiment, if M is a metal, the precursor of M comprises a metallic salt. In one embodiment, the metallic salt is a carboxylate of M, or a chloride of M, or a bromide of M, or a iodide of M, or a nitrate of M, or a sulfate of M, or a thiolate of M. According to one embodiment, the shell comprises a metal.

According to one embodiment, the shell to be deposited comprises a chalcogenide, a phosphide, a nitride, an arsenide or an oxide.

According to one embodiment, the initial nanosheet is dispersed in a solvent. According to one embodiment, the solvent is organic, preferably apolar or weakly polar. According to one embodiment, the solvent is a supercritical fluid or an ionic fluid. According to one embodiment, the solvent is selected from distilled water, methanol, ethanol, isopropanol, butanol, chloroform, acetone, hexane, tetrahydrofuran, dimethylsulfoxide, toluene, octadecene, squalane, trioctylamine, oleylamine, hexadecylamine, octadecylamine, squalene, and/or dimethylformamide.

According to one embodiment, the shell comprises an additional element in minor quantities. The term "minor quantities" refers herein to quantities ranging from 0.0001% to 10% molar, preferably from 0.001% to 10% molar.

According to one embodiment, the shell comprises a transition metal or a lanthanide in minor quantities. The term "minor quantities" refers herein to quantities ranging from 0.0001 % to 10% molar, preferably from 0.001 % to 10% molar.

According to one embodiment, the shell comprises in minor quantities an element inducing an excess or a defect of electrons compared to the sole film. The term "minor quantities" refers herein to quantities ranging from 0.0001% to 10% molar, preferably from 0.001% to 10% molar.

According to one embodiment, a reducing agent is introduced at the same time as at least one of the precursor of M and/or E. In one embodiment, the reducing agent comprises a hydride. Said hydride may be selected from sodium tetrahydroborate (NaBH4); sodium hydride (NaH), lithium tetrahydroaluminate (LiAlH4), diisobutylaluminum hydride (DIBALH). In one embodiment, the reducing agent comprises dihydrogen.

According to one embodiment, a stabilizing compound capable of stabilizing the final nanoplatelet is introduced in the solvent.

According to one embodiment, a stabilizing compound capable of stabilizing the final nanoplatelet is introduced in anyone of the precursor solutions.

According to one embodiment, the stabilizing compound of the final nanoplatelet comprises an organic ligand. Said organic ligand may comprise a carboxylic acid, a thiol, an amine, a phosphine, an amide, an ester, a pyridine, an imidazole and/or an alcohol.

According to one embodiment, the stabilizing compound of the final nanoplatelet is an ion. Said ion comprises a quaternary ammonium.

According to one embodiment, the initial nanosheet is fixed on a least one substrate.

According to one embodiment, the fixation of the initial nanosheet on said substrate is performed by adsorption or chemical coupling.

According to one embodiment, said substrate is chosen among silica SiO₂, aluminum oxide Al₂O₃, indium-tin oxide ITO, fluorine-doped tin oxide FTO, titanium oxide TiO₂, gold, silver, nickel, molybdenum, aluminum, silicium, germanium, silicon carbide SiC, graphene and cellulose.

According to one embodiment, said substrate comprises a polymer.

According to one embodiment, the excess of precursors is discarded after the reaction.

According to one embodiment, the final nanoplatelet obtained after reaction of the precursors on the initial nanosheets is purified. Said purification is performed by flocculation and/or precipitation and/or filtration; such as for example successive precipitation in ethanol.

The present invention also relates to a population of semiconductor nanoplatelets, each member of the population comprising a nanoplatelet core including a first semiconductor material and at least one shell including a second semiconductor material on the surface of the nanoplatelet core, wherein after ligand exchange reaction the population exhibits a quantum yield decrease of less than 50%.

According to one embodiment, the population of semiconductor nanoplatelets of the present invention exhibit, after ligand exchange, a quantum yield decrease of less than 50%, less than 40%, less than 30%, less than 25%, less than 20%, less than 15% or less than 10%.

Especially, according to one embodiment, after transfer into an aqueous solution by ligand exchange reaction, the quantum yield of the population of nanoplatelets according to the present invention decrease of less than 50%, less than 40%, less than 30%, less than 25%, less than 20%, less than 15% or less than 10%.

According to one embodiment, the ligand is an organic ligand with a carbonated chain length between 1 and 30 carbons.

According to one embodiment, the ligand is a polymer.

According to one embodiment, the ligand is a hydrosoluble polymer.

According to one embodiment, the selected ligand may comprise a carboxylic acid, a thiol, an amine, a phosphine, a phosphine oxide, an amide, an ester, a pyridine, an imidazole and/or an alcohol.

According to one embodiment, the ligand is selected from myristic acid, stearic acid, palmitic acid, oleic acid, behenic acid, dodecanethiol, oleylamine, 3-mercaptopropionic acid.

According to one embodiment, the selected ligand may be any number of materials, but has an affinity for the semiconductor surface. In general, the capping agent can be an isolated organic molecule, a polymer (or a monomer for a polymerization reaction), an inorganic complex, and an extended crystalline structure.

According to one embodiment, the ligand exchange procedure comprises the step of treating a solution of nanoplatelets according to the invention with a ligand.

The present invention also relates to a population of semiconductor nanoplatelets wherein the population exhibits stable fluorescence quantum efficiency over time. According to one embodiment, the population of nanoplatelets, wherein each member of the population comprising a nanoplatelet core including a first semiconductor material and a shell including a second semiconductor material on the surface of the nanoplatelet core, exhibits fluorescence quantum efficiency decrease of less than 50%, less than 40%, less than 30% after one hour under light illumination.

According to one embodiment, the light illumination is provided by blue or UV light source such as laser, diode or Xenon Arc Lamp.

According to one embodiment, the photon flux of the illumination is comprised between 1 mW.cm⁻² and 100 W.cm⁻² and more preferably between 10 mW.cm⁻² and 50 W.cm⁻², and even more preferably between 10 mW.cm⁻² and 10 W.cm⁻².

According to one embodiment, the population of nanoplatelets, wherein each member of the population comprising a nanoplatelet core including a first semiconductor material and a shell including a second semiconductor material on the surface of the nanoplatelet core, exhibits fluorescence quantum efficiency decrease of less than 80%, less than 70%, less than 60%, less than 50%, less than 40%, less than 30%, less than 20% or less than 15% after 2 months after a ligand exchange.

According to one embodiment, the semiconductor nanoplatelets of the invention exhibit enhanced stability in time compared to quantum dots and nanoplatelets of the prior art.

According to one embodiment, the semiconductor nanoplatelets of the invention exhibit enhanced stability in temperature compared to quantum dots and nanoplatelets of the prior art.

According to one embodiment, the core/shell nanoplatelets according to the present invention exhibit stable fluorescence quantum efficiency in temperature. According to one embodiment, the population of semiconductor nanoplatelets according to the invention exhibits fluorescence quantum efficiency at 100 °C or above that is at least 50%, at least 60%, at least 70%, at least 80%, or at least 90% of the fluorescence quantum efficiency of the population at 20°C. According to one embodiment, the temperature is in a range from 100°C to 250°C, from 100°C to 200°C, from 110°C to 160°C or about 140°C. According to one embodiment, the population of semiconductor nanoplatelets according to the invention exhibits fluorescence quantum efficiency at 200°C that is at least 50%, at least 60%, at least 70%, at least 80% or at least 90% of the fluorescence quantum efficiency of the population at 20°C.

According to one embodiment, the population of nanoplatelets according to the present invention exhibit emission spectra with a full width half maximum lower than 50, 40, 30, 25 nm or 20 nm.

The present invention also relates to a population of fluorescent colloidal nanoplatelets, each member of the population comprising a nanoplatelet core including a first semiconductor material and a shell including a second semiconductor material on the surface of the nanoplatelet core, wherein at least 40% of the nanoplatelets of the population are continuously emissive for a period of at least one minute.

According to one embodiment, at least 40%, at least 50%, at least 60%, at least 70%, at least 75%, at last 80% or at least 90% of the nanoplatelets of the population are continuously emissive for a period of at least one minute.

According to one embodiment, the shell of the nanoplatelets of the population has a thickness of at least 1 nm, at least 3 nm, at least 5 nm, at least 5.5 nm, at least 6 nm, at least 7 nm, at least 8 nm, at least 8.5 nm, at least 9 nm, at least 10 nm.

According to one embodiment, as depicted in figure 10, at least 40% of a population of core/shell nanoplatelets having a shell with a thickness of at least 3 nm is continuously emissive for a period of at least one minute. According to one embodiment, as depicted in figure 12, at least 85% of a population of core/shell nanoplatelets having a shell with a thickness of at least 5.5 nm is continuously emissive for a period of at least one minute. According to one embodiment, as depicted in figure 15, at least 90% of a population of core/shell nanoplatelets having a shell with a thickness of at least 8 nm is continuously emissive for a period of at least one minute.

The present invention also relates to the use of the final nanoplatelets described above. Indeed, these final nanoplatelets further grown in the thickness have numerous advantages and properties.

These properties, especially the suppressed blinking make the final nanoplatelets useful objects for applications as fluorophore.

These different properties make also the final nanoplatelets useful objects for applications in fluorescence, especially in luminescent or electroluminescent systems or in the amplification stage of a laser due to their narrow FWMH, their high quantum yield, their resistance to photobleaching, their resistance to light flux and their fluorescence stability over temperature variations, their resistance to ligand exchange, their resistance to surface chemistry modifications.

As an example, CdSe nanosheets covered with a CdS or a CdZnS shell as described in the examples have a high quantum yield that can be over 80% as well as a narrow fluorescence full width at half maximum (less than 23 nm). Other examples of final particles or objects useful for applications in fluorescence are CdS nanosheets covered with ZnS (CdS/ZnS), CdSeₓS₁₋ₓ (x being a decimal number from 0 to 1) covered with Cd_{y}Zn_{1-y}S (y being a decimal number from 0 to 1) (CdSeS/CdZnS), CdTe nanosheets covered with Cd_{y}Zn_{1-y}SeₓS₁₋ₓ (x and y being decimal number independently from 0 to 1) (CdTe/CdZnSeS), CdS nanosheets covered with manganese(II) or copper(II)-doped ZnS (CdS/ZnS:Mn or CdS/ZnS:Cu).

The final nanoplatelets are also useful as catalysts because of their high specific surface, their better stability, their great absorption cross-section.

CdS, ZnS and alloys thereof nanosheets and CdSe nanosheets can for example be covered with a shell of platinum and/or palladium sulfide. The obtained composite has a photocatalytic dihydrogen production activity. Other examples of final particles or objects useful in catalytic applications are CdS or CdSe nanosheets covered with TiO₂, CdSe or CdS nanosheets covered with a metallic shell such as Cu, Ag, Au, Ni, Pd, Pt, Co, Fe, Ru, Os, Mn, Tc, Re, Cr, Mo, W, V, Nd, Ta, Ti, Zr, Hf.

The final nanoplatelets are also very useful as an absorbing and/or collecting element of a photovoltaic cell because of their better stability, enhanced electron/hole mobility and high absorption cross section.

As an example, CdSe nanosheets with big dimensions (more than 100 nm of lateral extension) can be covered with a doped shell (CdZnS doped with Ag⁺ for example). This allows increasing the electronic mobility of the absorbing and collecting elements of a solar cell containing such final object: CdSe/CdTe, CdTe/CdSe, PbSe/PbS, ZnS/InP, CuInS2/ZnS, CdS/CuInS₂.

The final nanoplatelets are also useful as field effect transistor due to their better stability, enhanced electron/hole mobility and a high density of charge carriers.

As an example, CdSe nanosheets with big dimensions (more than 100 nm of lateral extension) can be covered with a doped shell (CdZnS doped with Ag⁺ for example). This allows enhancing the electronic mobility in the final objects. Equally, a low band gap semiconducting sheet covered with a high band gap semiconductor shell allows obtaining a high mobility while keeping an excitonic structure. PbS nanosheets covered with a layer of CdS is an example of final particles or objects useful for field effect transistor applications.

The final nanoplatelets are also useful as phosphors for down-converting light applications, thanks to their high fluorescence quantum yield, their resistance to photobleaching, their resistance to light flux and their fluorescence stability over temperature variations.

As an example, core/shell nanoplatelets such as CdSe/ZnS can be introduced into a polymer to prepare a polymer-nanoplatelets composite film. Such composite films absorb efficiently blue light generated by a primary GaN diode and convert it into light of longer wavelength.

Another aspect of the invention relates to a luminescent system, an electroluminescent system, an amplifying stage of a laser, a high specific surface and crystallinity catalyst of controlled surface, an absorbing and/or collecting element of a photovoltaic cell or a field effect transistor comprising a nanoplatelet according to the invention.

The present invention also relates to nanoplatelets film exhibiting desirable characteristics for use in display devices, such as narrow full width at half maximum, high quantum yield and resistance to photo-bleaching.

According to one embodiment, the nanoplatelets film comprises a host material, preferably a polymeric host material and emissive semiconductor nanoparticles embedded in said host material, wherein at least 20% of said emissive semiconductor nanoparticles are colloidal nanoplatelets according the invention.

In one embodiment, at least 20%, 30%, 40%, 50%, 60%, 70%, 80%, 90% of said emissive semiconductor nanoparticles are colloidal core/shell nanoplatelets according to the present invention. In one embodiment, substantially all of said emissive semiconductor nanoparticles are colloidal core/shell nanoplatelets according to the present invention.

In one embodiment the nanoplatelets film comprises less than 50% in weight of emissive semiconductor nanoparticles, preferentially less than 10%.

In one embodiment the nanoplatelets film has a thickness between 30nm and 1cm, more preferably between 100nm and 1mm, even more preferably between 100nm and 500µm.

In one embodiment, the nanoplatelets film refers to a layer, sheet or film of host material that comprises a plurality of nanoplatelets.

In one embodiment, the nanoplatelets comprise an outer ligand coating and are dispersed in the host material, preferably a polymeric host material. In on embodiment, the host material is transparent in the visible range of wavelength.

In one embodiment the polymeric host material used to include the nanoplatelets is chosen among: silicone-based polymers, polydimethylsiloxanes (PDMS), polyethylene terephthalate (PET), polyesters, polyacrylates, poly(methacrylates), polycarbonate, polyvinyl alcohol, polyvinylpyrrolidone, polysaccharides, melamine resins, a phenol resin, an alkyl resin, an epoxy resin, a polyurethane resin, a maleic resin, a polyamide resin, an alkyl resin, a maleic resin, copolymers forming the resins, polymerizable monomers, and comprising an UV initiator.

In one embodiment the polymeric host material used to include the nanoplatelets is a polymerized solid made from an alkyl methacrylates or an alkyl acrylates such as acrylic acids and derivatives for example, acrylic acid, methacrylic acid, crotonic acid, acrylonitrile, acrylic esters substituted with methoxy, ethoxy, propoxy, butoxy, and similar derivatives for example, methyl acrylate, propyl acrylate, butyl acrylate, methyl methacrylate, methyl crotonate, glycidyl methacrylate, alkyl crotonates, and related esters.

In one embodiment the polymeric host material used to include the nanoplatelets comprises PMMA, Poly(maleic anhydride -alt-octadecene).

In one embodiment the polymeric host material used to include the nanoplatelets is a polymerized solid made from allyl methacrylate, benzyl methyl acrylate, 1,3- butanediol dimethacrylate, 1,4-butanediol dimethacrylate, butyl acrylate, n-butyl methacrylate, ethyl methacrylate, 2-ethyl hexyl acrylate, 1,6-hexanediol dimethacrylate, 4- hydroxybutyl acrylate, hydroxyethyl acrylate, 2-hydroxyethyl methacrylate, 2- hydroxypropyl acrylate, isobutyl methacrylate, lauryl methacrylate, methacrylic acid, methyl acrylate, 2,2,3,3 ,4,4,5,5-octafluoropentyl acrylate, pentaerythritol triacrylate, 2,2,2- trifluoroethyl 2-methyl acrylate, trimethylolpropane triacrylate, acrylamide n,n,-methylene- bisacrylamide phenyl acrylate, and divinyl benzene.

In one embodiment the polymeric host material used to include the nanoplatelets is a polymerized solid made from alpha- olefins; dienes such as butadiene and chloroprene; styrene, alpha-methyl styrene, and the like; heteroatom substituted alpha-olefins, for example, vinyl acetate, vinyl alkyl ethers for example, ethyl vinyl ether, vinyltrimethylsilane, vinyl chloride, tetrafluoroethylene, chlorotrifiuoroethylene, N-(3-dimethylammopropyl methacrylamide, dimethylammopropyl methacrylamide, acrylamide, methacrylamide, and similar derivatives; cyclic and polycyclic olefin compounds for example, cyclopentene, cyclohexene, cycloheptene, cyclooctene, and cyclic derivatives up to C2o; polycyclic derivates for example, norbornene, and similar derivatives up to C2o; cyclic vinyl ethers for example, 2, 3-dihydrofuran, 3,4-dihydropyran, and similar derivatives; allylic alcohol derivatives for example, vinylethylene carbonate, disubstituted olefins such as maleic and fumaric compounds for example, maleic anhydride, diethylfumarate, and the like; and mixtures thereof.

In one embodiment the polymeric host material used to include the nanoplatelets is deposited under its final form tanks to spincoating, dipcaoting, electrophoretic deposition, dropcasting. In one embodiment the polymeric host material is mixed with the nanoplatelets thanks to an extrusion process.

According to one embodiment, the nanoplatelets film further comprises scattering elements dispersed in the host material.

In one embodiment, the nanoplatelets film comprises at least one population of nanoplatelets. In the present application a population of nanoplatelets is defined by the maximum emission wavelength.

In one embodiment the nanoplatelets film comprises two populations of nanoplatelets with different colors. In one embodiment, the nanoplatelets film consists of nanoplatelets which emit green light and red light upon down-conversion of a blue light source. Thus, the blue light from the light source(s) pass through the nanoplatelets film, where predetermined amounts of green and red light are mixed with the remaining blue light to create the tri-chromatic white light.

In one embodiment, the nanoplatelets film comprises two populations of nanoplatelets, a first population with a maximum emission wavelength between 500 nm and 560 nm, more preferably between 515 nm and 545 nm and a second population with a maximum emission wavelength between 600 nm and 700 nm, more preferably between 610 nm and 650 nm.

In one embodiment the nanoplatelets film comprises two populations of core/shell nanoplatelets with different color. In one embodiment the nanoplatelets film comprises two populations of core/shell nanoplatelets one is green and one is red, see figure 5.

In one embodiment the nanoplatelets films comprises a blend of two populations of core/shell nanoplatelets with different colors.

In one embodiment the nanoplatelets films is splitted in several area each of them comprise a different population having different color of core/shell nanoplatelets.

In one embodiment the nanoplatelets film is made of a stack of two films, each of them comprises a different population of nanoplatelets having a different color.

In on embodiment, the nanoplatelets film is encapsulated into a multi-layered system. In one embodiment, the encapsulated nanoplatelets film is made of at least three layers. According to one embodiment, the two external layers provide scattering properties.

In one embodiment, the nanoplatelets film is covered by at least one insulating layer or sandwiched by at least two insulating layers, see figure 1. In one embodiment, the nanoplatelets film is enclosed in an O₂ and/or H₂O non-permeable layer. In one embodiment, the O₂ and/or H₂O insulating layer can be made of glass, PET, PDMS... According to one embodiment, the nanoplatelets film is enclosed in a layer configured to reduce exposure of the nanoplatelets film to O₂ and H₂O, such as glass, PET, PDMS... In one embodiment, the encapsulated nanoplatelets film also comprises at least one transparent substrate.

In one embodiment the polymer host material comprising the nanoplatelets is protected from air by an additional layer. In one embodiment the polymer host material comprising the nanoplatelets is protected from air by UV curable polymer. In one embodiment the host material comprising the nanoplatelets is protected from air by UV curable resin. In one embodiment the polymer host material comprising the nanoplatelets is protected from air by a mixture of bisphenol A glycerolate, lauryl methacrylate and an UV initiator such as benzophenone or 3,4 dimethylbenzophenone.

Other characteristics and advantages of the nanoplatelets and process for their manufacture according to the invention will appear after reading the examples given after as purely illustrative means.

### BRIEF DESCRIPTION OF THE DRAWINGS

**Figure 1** shows a) an absorption spectrum and b) an emission spectrum of CdSe/CdS core/shell nanoplatelets according to the present invention.
**Figure 2A** and **2B** show TEM images of CdSe/CdS core/shell nanoplatelets with a shell thickness of 1.5 nm according to the present invention.

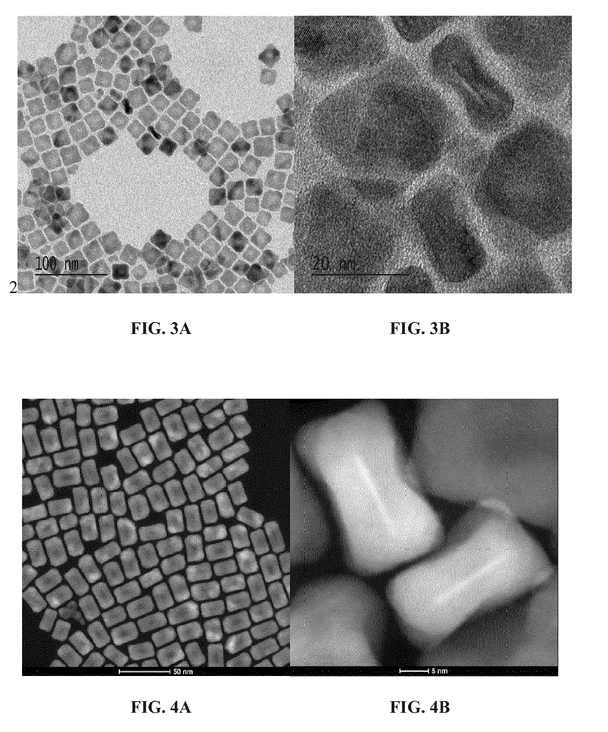

**Figure 3A** and **3B** show TEM images of CdSe/CdS core/shell nanoplatelets with a shell thickness of 3 nm according to the present invention.
**Figure 4A** and **4B** show TEM images of CdSe/CdS core/shell nanoplatelets with a shell thickness of 5.5 nm according to the present invention.
**Figure 5** shows of a) an absorption spectrum and b) an emission spectrum of CdSe/CdZnS core/shell nanoplatelets according to the present invention.
**Figure 6** shows TEM images of CdSe/CdZnS core/shell nanoplatelets according to the present invention.

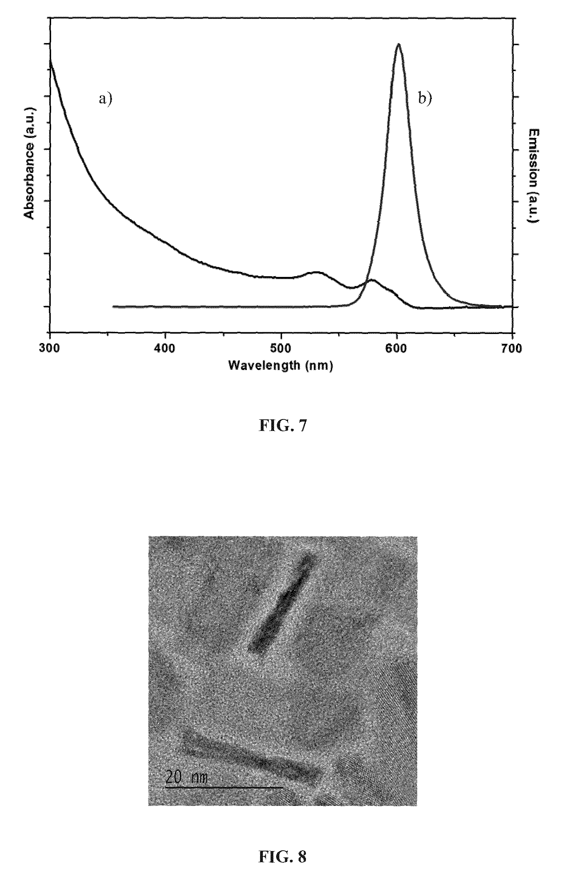

**Figure 7** shows a) an absorption spectrum and b) an emission spectrum of CdSe/ZnS core/shell nanoplatelets according to the present invention.
**Figure 8** shows TEM images of CdSe/ZnS core/shell nanoplatelets according to the present invention.

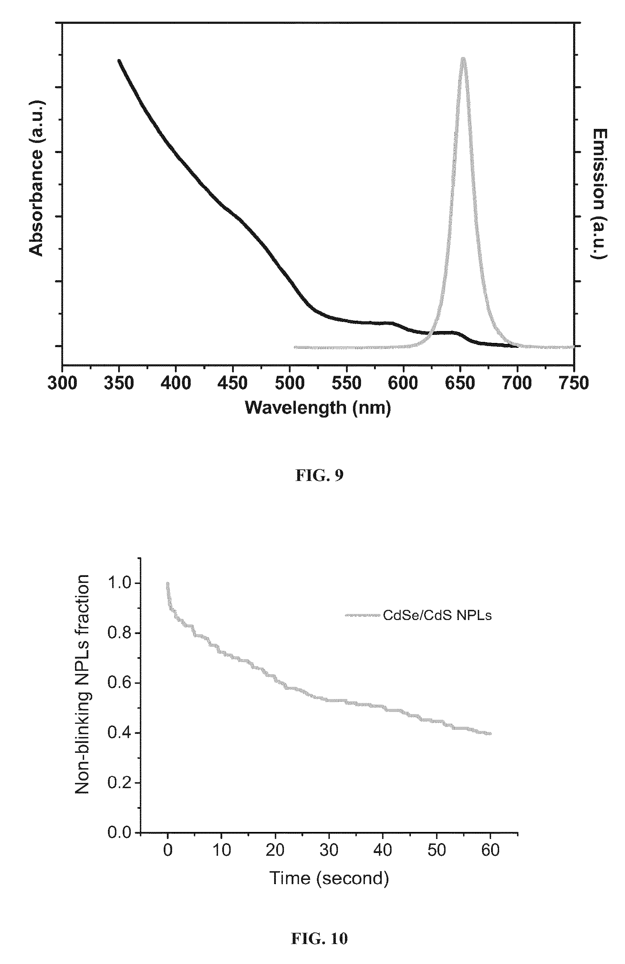

**Figure 9** shows the evolution of the absorption spectrum (black) and the emission spectrum (grey) of CdSe/CdZnS core/shell nanoplatelets according to the present invention after ligand exchange with a hydrosoluble polymer.
**Figure 10** shows the non-blinking fraction of CdSe/CdS core/shell nanoplatelets with a shell thickness of 3 nm according to the present invention (i.e. the fraction of continuously emissive nanoplatelets).

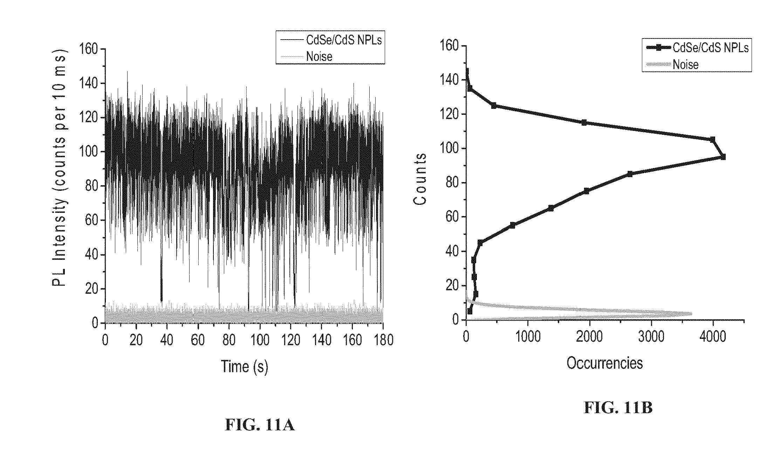

**Figures 11A** and **11B** show the emission time trace and corresponding normalized fluorescence intensity distribution of a single CdSe/CdS core/shell nanoplatelets with a shell thickness of 3 nm according to the present invention (trace in black and background noise in grey).

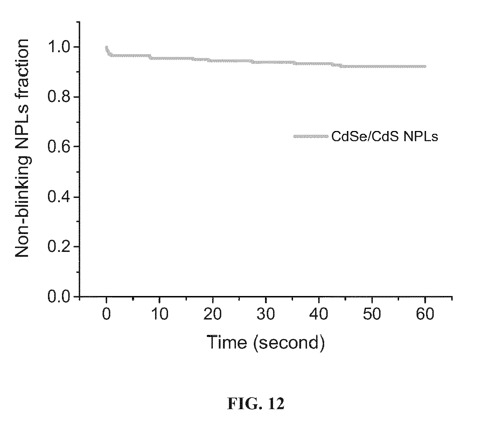

**Figure 12** shows the non-blinking fraction of CdSe/CdS core/shell nanoplatelets with a shell thickness of 5.5 nm according to the present invention (i.e. the fraction of continuously emissive nanoplatelets).

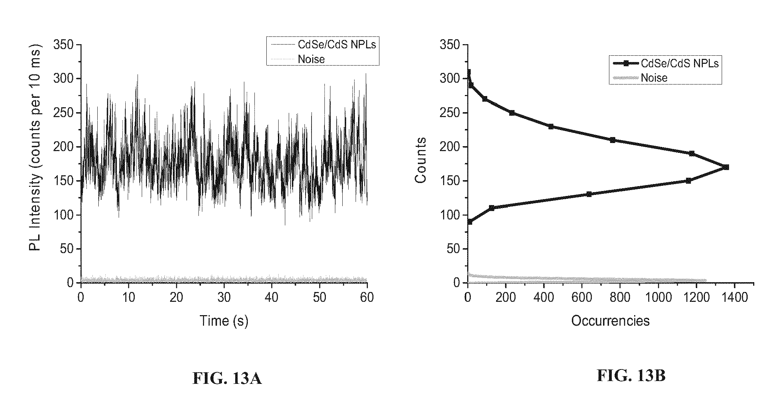

**Figures 13A** and **13B** show the emission time trace and corresponding normalized fluorescence intensity distribution of a single CdSe/CdS core/shell nanoplatelets with a shell thickness of 5.5 nm according to the present invention (trace in black and background noise in grey).

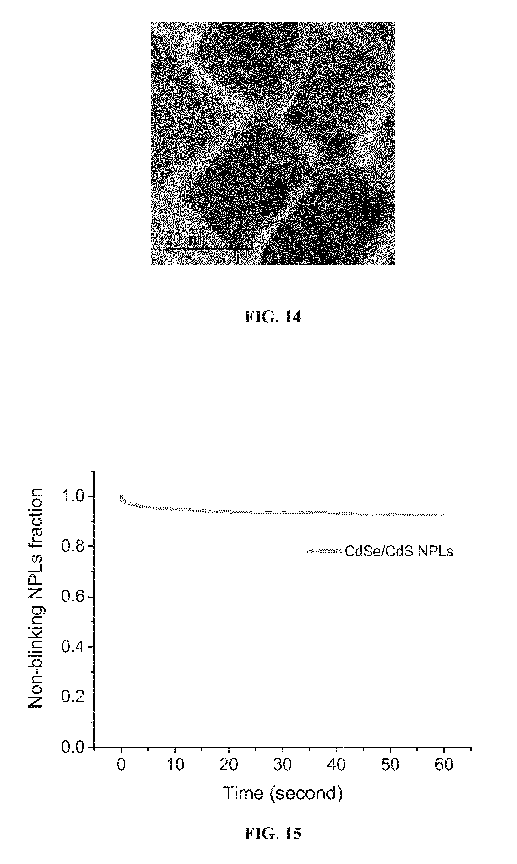

**Figure 14** shows TEM images of CdSe/CdS core/shell nanoplatelets with a shell thickness of 8.5 nm according to the present invention.
**Figure 15** shows the non-blinking fraction of CdSe/CdS core/shell nanoplatelets with a shell thickness of 8.5 nm according to the present invention (i.e. the fraction of continuously emissive nanoplatelets).

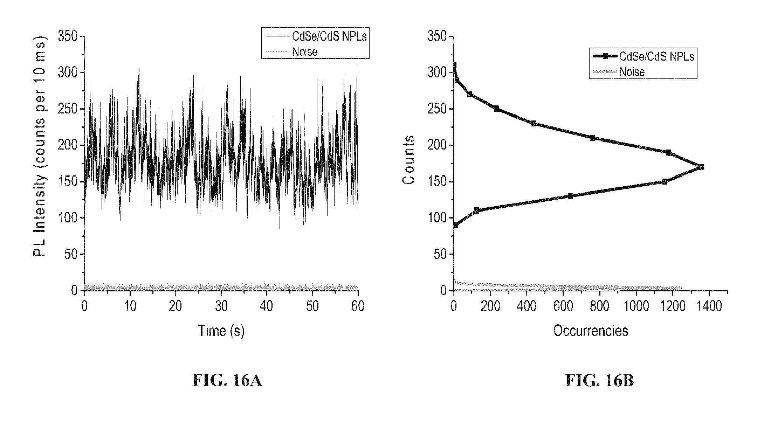

**Figures 16A** and **16B** show the emission time trace and corresponding normalized fluorescence intensity distribution of a single CdSe/CdS core/shell nanoplatelets with a shell thickness of 8.5 nm according to the present invention (trace in black and background noise in grey).
**Figure 17** shows the measurement of the normalized fluorescence quantum efficiency coming from film of CdSe/CdZnS nanoplatelets according to the invention, quantum dots of the prior art or CdSe/CdZnS nanoplatelets of the prior art deposed on microscope glass slides. Films are excited using a Hg lamp, and the emitted light is collected with an oil objective (100x, NA = 1.4) and adapted filters (550 nm short-pass filter for the excitation and 590 nm long-pass filter for the emission).
**Figure 18** shows the measurement of the normalized fluorescence quantum efficiency coming from CdSe/ZnS nanoplatelets according to the invention, CdSe/CdZnS nanoplatelets according to the invention, CdSe/CdS/ZnS quantum dots according to the prior art and CdSe/CdZnS nanoplatelets according to the prior art deposed on a glass slide in function of temperature. Films are excited with a laser at 404 nm.

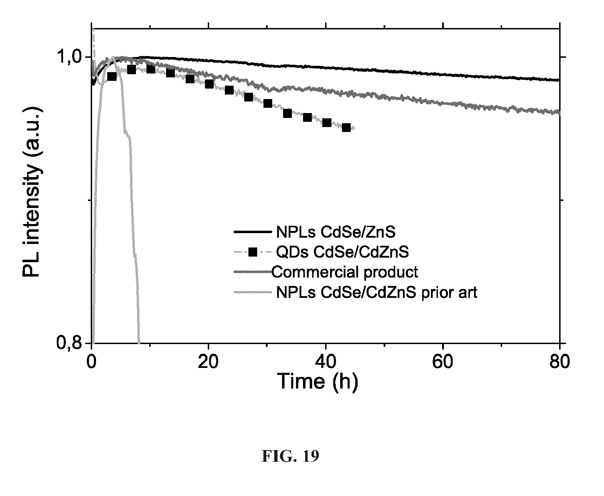

**Figure 19** shows the measurement of the normalized fluorescence quantum efficiency coming from a layered material comprising CdSe/CdZnS nanoplatelets of the invention, quantum dots of the prior art or CdSe/CdZnS nanoplatelets of the prior art under blue LED excitation operated at 200mA, (see photobleaching measurements after encapsulation).

### EXAMPLES

### Nanoplatelets cores preparations

### Synthesis of CdSe 460 nanoplatelets (NPLs)

240 mg of Cadmium acetate (Cd(OAc)₂) (0,9mmol), 31 mg of Se 100 mesh, 150µL oleic acid (OA) and 15mL of 1-octadecene (ODE) are introduced in a three neck flask and are degassed under vacuum. The mixture is heated under argon flow at 180°C for 30 min.

### Synthesis of CdSe 510 NPLs

170mg of cadmium myristate (Cd(myr)₂) (0,3mmol), 12mg of Se 100 mesh and 15mL of ODE are introduced in a three neck flask and are degassed under vacuum. The mixture is heated under argon flow at 240°C, when the temperature reaches 195°C, 40mg of Cd(OAc)₂ (0,15mmol) are introduced. The mixture is heated for 10 minutes at 240°C.

### Synthesis of CdSe 550 NPLs

170mg of Cd(myr)₂ (0,3mmol) and 15mL of ODE are introduced in a three neck flask and are degassed under vacuum. The mixture is heated under argon flow at 250°C and 1mL of a dispersion of Se 100 mesh sonicated in ODE (0,1M) are quickly injected. After 30 seconds, 80mg of Cd(OAc)₂ (0,3mmol) are introduced. The mixture is heated for 10 minutes at 250°C.

### Synthesis of CdTe 428 NPLs

A three neck flask is charged with 130 mg of cadmium proprionate (Cd(prop)₂) (0.5 mmol), 80 µL of OA (0.25 mmol), and 10 mL of ODE, and the mixture is stirred and degassed under vacuum at 95°C for 2 h. The mixture under argon is heated at 180°C and 100 µL of a solution of 1 M Te dissolved in trioctylphosphine (TOP-Te) diluted in 0.5 mL of ODE are swiftly added. The reaction is heated for 20 min at the same temperature.

When 428 NPLs are prepared using Cd(OAc)₂, TOP-Te 1 M is injected between 120 and 140°C.

### Synthesis of CdTe 500 NPLs

A three-neck flask is charged with 130 mg of Cd(prop)₂ (0.5 mmol), 80 µL of OA (0.25 mmol), and 10 mL of ODE, and the mixture is stirred and degassed under vacuum at 95°C for 2 h. The mixture under argon is heated at 210°C, and 100 µL of a solution of 1 M TOP-Te diluted in 0.5 mL of ODE is swiftly added. The reaction is heated for 30 min at the same temperature.

When Cd(OAc)2 was used as cadmium precursor, TOP-Te is injected between 170 and 190°C.

### Synthesis of CdTe 556 NPLs

133 mg of Cd(OAc)₂ (0.5 mmol), 255 µL of OA (0.8 mmol), and 25 mL of ODE are charged into a three-neck flask, and the mixture is stirred and degassed under vacuum at 95°C for 2 h. The flask is filled with argon and the temperature is increased to 215°C. Then, 0.05 mmol of stoichiometric TOP-Te (2.24 M) diluted in 2.5 mL ODE is injected with a syringe pump at a constant rate over 15 min. When the addition is completed, the reaction is heated for 15 min.

### Synthesis of CdS 375 NPLs

In a three neck flask 160 mg of Cd(OAc)₂ (0.6mmol), 190 µL (0.6mmol) of OA, 1.5 mL of sulfur dissolved in 1-octadecene (S-ODE) 0.1M and 13.5 mL of ODE are introduced and degassed under vacuum for 30 minutes. Then the mixture is heated at 180°C under Argon flow for 30 minutes.

### Synthesis of CdS 407 NPLs

In a three neck flask 160 mg of Cd(OAc)₂ (0.6mmol), 190 µL (0.6mmol) of OA, 1.5 mL of S-ODE 0.1M and 13.5 mL of octadecene are introduced and degassed under vacuum for 30 minutes. Then the mixture is heated at 260°C under Argon flow for 1 minute.

### Synthesis of Core/Shell (Crown) or shell CdSe/CdS NPLs

In a three neck flask, 320 mg of Cd(OAc)₂ (1.2 mmol), 380 µL of OA (1.51 mmol) and 8 mL of octadecene are degassed under vacuum at 65°C for 30 minutes. Then CdSe nanoplatelets cores in 4 mL of ODE are introduced under Argon. The reaction is heated at 210 °C and 0.3 mmol of S-ODE 0.05M are added drop wise. After injection, the reaction is heated at 210°C for 10 minutes.

### Synthesis of Core/Shell (Crown) CdSe/CdTe NPLs

In a three neck flask, CdSe nanoplatelets cores in 6 mL of ODE are introduced with 238 µL of OA (0.75 mmol) and 130 mg of Cd(prop)₂. The mixture is degassed under vacuum for 30 minutes then, under argon, the reaction is heated at 235°C and 50 µL of TOP-Te 1M in 1 mL of ODE is added drop wise. After the addition, the reaction is heated at 235°C for 15 minutes.

### Synthesis of CdSeS alloyed NPLs

170mg of Cd(myr)₂ (0.3 mmol) and 15mL of ODE are introduced in a three neck flask and are degassed under vacuum. The mixture is heated under argon flow at 250°C and 1mL of a dispersion of Se 100 mesh sonicated in S-ODE and ODE (total concentration of selenium and sulfur 0.1M) are quickly injected. After 30 seconds, 120mg of Cd(OAc)₂ (0.45 mmol) are introduced. The mixture is heated for 10 minutes at 250°C.

### Shells growth

### CdS shell growth

In a three neck flask, 15 mL of trioctylamine (TOA) are introduced and degassed under vacuum at 100°C. Then the reaction mixture is heated at 300°C under Argon and 5 mL of core nanoplatelets in ODE are swiftly injected followed by the injection of 7 mL of 0.1 M octanethiol solution in ODE and 7 mL of 0.1 M Cd(OA)₂ in ODE with syringe pumps at a constant rate over 90 min. After the addition, the reaction is heated at 300°C for 90 minutes.

### ZnS shell growth

In a three neck flask, 15 mL of trioctylamine are introduced and degassed under vacuum at 100°C. Then the reaction mixture is heated at 300°C under Argon and 5 mL of core nanoplatelets in octadecene are swiftly injected followed by the injection of 7 mL of 0.1 M octanethiol solution in octadecene and 7 mL of 0.1 M zinc oleate (Zn(OA)₂) in octadecene with syringe pumps at a constant rate over 90 min. After the addition, the reaction is heated at 300°C for 90 minutes.

### CdZnS gradient shell growth

In a three neck flask, 15 mL of trioctylamine are introduced and degassed under vaccum at 100°C. Then the reaction mixture is heated at 300°C under Argon and 5 mL of core nanoplatelets in octadecene are swiftly injected followed by the injection of 7 mL of 0.1 M octanethiol solution in octadecene with syringe pumps at a constant rate and 3.5 mL of 0.1 M Cd(OA)₂ in octadecene and 3.5 mL of 0.1 M Zn(OA)₂ in octadecene with syringe pumps at variables rates over 90 min. After the addition, the reaction is heated at 300°C for 90 minutes.

### CdZnS alloys shell growth

In a three neck flask, 15 mL of trioctylamine are introduced and degassed under vaccum at 100°C. Then the reaction mixture is heated at 300°C under Argon and 5 mL of core nanoplatelets in octadecene are swiftly injected followed by the injection of 7 mL of 0.1 M octanethiol solution in octadecene, 3.5 mL of 0.1M Cd(OA)₂ in octadecene and 3.5 mL of 0.1M Zn(OA)₂ in octadecene with syringe pumps at a constant rate over 90 min. After the addition, the reaction is heated at 300°C for 90 minutes.

### CdZnS shell growth (manufacture according to the prior art: ambient temperature. Mahler et al. JACS. 2012, 134(45), 18591-18598)

1 mL of CdSe 510 NPLs in hexane is diluted in 4 mL of chloroform, then 100 mg of thioacetamide (TAA) and 1 mL of octylamine are added in the flask and the mixture is sonicated until complete dissolution of the TAA (about 5 min). The color of the solution changed from yellow to orange during this time. 350 µL of a solution of Cd(NO₃)₂ 0.2 M in ethanol and 150 µL of a solution of Zn(NO₃)₂ 0.2 M in ethanol are then added to the flask. The reaction was allowed to proceed for 2 h at 65°C. After synthesis, the core/shell platelets were isolated from the secondary nucleation by precipitation with a few drops of ethanol and suspended in 5 mL of chloroform. Then 100 µL of Zn(NO₃)₂ 0.2 M in ethanol is added to the nanoplatelets solution. They aggregate steadily and are resuspended by adding 200 µL oleic acid.

### ZnS alternative shell growth

In a three neck flask, 15 mL of trioctylamine are introduced and degassed under vacuum at 100°C. Then the reaction mixture is heated at 310°C under Argon and 5 mL of core nanoplatelets in octadecene mixed with 50µL of precursors mixture are swiftly injected followed by the injection of 2 mL of 0.1M zinc oleate (Zn(OA)₂) and octanethiol solution in octadecene with syringe pump at a constant rate over 80 min.

### Effect of ligand Exchanged on Quantum Yield

### Ligand exchange procedure (1-dodecanethiol)

Ligand exchange with 1-dodecanthiol have been done by treating 1 mL of core/shell nanoplatelets solution with 200 µL of 1-dodecanethiol. Then the solution is left without stirring at 65°C overnight. After, the exchanged nanoplatelets are washed by two successive precipitations by EtOH and resuspension in hexane (Table 1).

**Table 1**

| **Sample** | **Native NPLs** | **After ligand exchange¹** |
|---|---|---|
| NPLs CdSe/CdZnS | 75% | 69% |
| NPLs CdSe/CdS/ZnS | 72% | 65% |
| NPLs CdSe/ZnS | 74% | 72% |
| NPLs CdSe/CdZnS of the prior art | 58% | 10% |

| | | |
|---|---|---|
| ¹ Exchanged with 1-dodecanethiol. | | |

### Ligand exchange procedure (polymerized ligands)

1 mg of Core/shell nanoplatelets in hexane are precipitated with ethanol and centrifuged. The supernatant is removed and the nanoplatelets are dispersed in 200 µL of 3-mercaptopropionic acid (MPA). The mixture is sonicated to obtain a homogenous dispersion. The nanoplatelets dispersion is stored at 60°C for 2 hours. Then the nanoplatelets are centrifuged and the MPA phase is discarded. The nanoplatelets are dispersed in DMF under sonication and 2mg of potassium *tert*-butoxide are added and nanoplatelets dispersion is sonicated. The mixture is centrifuged and the DMF phase is discarded. The precipitated nanoplatelets are washed with ethanol and the nanoplatelets are dispersed in sodium tetraborate buffer. 200µL of aqueous solution of polymerized ligands, previously reduced 30 min with NaBH₄, are added to nanoplatelets dispersion. The solution is stored at 60°C overnight. The excess of free ligand and reagents are removed by Vivaspin. The evolution of the absorption spectrum and the emission spectrum of CdSe/CdZnS core/shell nanoplatelets after ligand exchange with a hydrosoluble polymer are for example shown in Figure 9. In addition, Table 2 exhibits the percentage (%) of Quantum yield on NPLs exchanged with polymerized ligands the following day and after 2 months.

**Table 2**

| **Sample** | **Native NPLs** | **After ligand exchange¹** | **2 month after ligand exchange²** |
|---|---|---|---|
| NPLs of the prior art | 20% | 7% | N.A. |
| NPLs CdSe/CdZnS | 75% | 69% | 62% |
| NPLs CdSe/ZnS | 68% | 65% | 60% |

| | | | |
|---|---|---|---|
| ¹ Exchanged with polymerized ligands. ² Stored at 4°C in the dark in a concentration of 10 µM. | | | |

### Layered material preparation:

A solution of CdSe-ZnS nanoplatelets is first precipitated in air free glove box by addition of ethanol. After centrifugation the formed pellet is redispersed in chloroform solution. Meanwhile a solution at 30 % in weight of Poly(maleic anhydride -alt-octadecene) (MW=40kg.mol-1) in chloroform is prepared. Then the nanoplatelets solution is mixed with the polymer solution in a 1:1 volume ratio and the solution is further stirred. On an 02 insulating substrate (glass or PET) the solution nanoplatelets-polymer mixture is brushed and let dried for 30min. Then UV polymerizable oligomer made of 99% of lauryl methacrylate and 1% of benzophenone is deposited on the top of the nanoplatelets film. A top substrate (same as the bottom substrate) is deposited on the system. The film is the polymerized under UV for 4 min. The layered material is then glued thanks to a PMMA solution dissolved in chloroform on a 455nm LED from Avigo technology. The LED is operated under a constant current ranging from 1mA to 500mA.

**Ensemble measurements:** The nanoplatelets in hexane solution are diluted in a mixture of 90% hexane/10% octane and deposited by drop-casting on a glass substrate. The sample is visualized using an inverted fluorescent microscope. An area of the sample containing several nanoplatelets is excited using a Hg lamp, and the emitted light is collected with an oil objective (100x, NA = 1.4) and adapted filters (550 nm short-pass filter for the excitation and 590 nm long-pass filter for the emission). The emitted light of the sample can be observed on a CCD camera (Cascade 512B, Roper Scientific) or directly through the microscope eyepiece with the naked eyes. A movie of the illuminated field containing at least 100 nanoplatelets is recorded for 1 minute at 33Hz frame rate. Using a home-made software, the fluorescence intensity time traces of the emitting nanoplatelets are extracted as well as the noise of the background. By fixing an "off" threshold at 3 times the noise, the time of the first "off" event is computed for each time trace. Plotting the number of nanoplatelets which never went "off" over time give access to the global Non-blinking fraction of nanoplatelets over time.

**Single particle measurements:** The fluorescence intensity of unique nanoplatelets are recorded on a confocal microscope (Microtime 200, Picoquant) and a Hanbury Brown and Twist setup based on two avalanche photodiodes (SPAD PDM, MPD, time resolution 50 ps). The photodetection signal is recorded by a HydraHarp 400 module (Picoquant). In this configuration, the studied nanocrystal is excited with a pulsed diode emitting at 405 nm. To obtain a single nanoplatelet spectrum, a part or the totality of the collected photons is sent to an Andor shamrock 750 spectrometer. The dispersion system is a prism, and the detector is a CCD camera (Cascade 512B, Roper Scientific). Typical time traces of single nanoplatelets are obtained by integrating the number of photons collected over 10 ms.

### Photobleaching measurements in air

The nanoplatelets or quantum dots in hexane solution are diluted in a mixture of 90% hexane/10% octane and deposited by drop-casting on a glass substrate. The sample is visualized using an inverted fluorescent microscope. An area of the sample containing nanoplatelets or quantum dots as a concentration still allowing distinguishing single nanocrystals is excited using a Hg lamp, and the emitted light is collected with an oil objective (100x, NA = 1.4) and adapted filters (550 nm short-pass filter for the excitation and 590 nm long-pass filter for the emission). The emitted light of the sample can be observed on a CCD camera (Cascade 512B, Roper Scientific). An image of the illuminated field is taken every minute and the mean intensity of the film is normalized with the initial intensity, allowing to plot the mean intensity variations over time (see figure 17).

### Fluorescence stability versus temperature measurement

The layered material preparation is described above. The layered material is heated via a hot plate at the desired temperature ranging from 20°C to 200°C and the fluorescence is measured using an optical fiber spectrometer (Ocean-optics usb 2000) under excitation with a laser at 404nm. The measurements are taken after temperature stabilization (see figure 18).

### Photobleaching measurements after encapsulation

The layered material glued to a LED as described above is excited using the LED emission under 200mA operation. The fluorescence of the layered material as well as a fraction of the blue light from the LED is acquired using an optical fiber spectrometer (Ocean-optics usb 2000). The stability of the fluorescence over time is obtained by normalizing the integrated fluorescence from the layered material by the integrated fluorescence from the blue LED. This fluorescence quantum efficiency is then normalized to the initial ratio and plotted over time for direct comparisons purposes (Figure 19).

## Claims

1. A population of semiconductor nanoplatelets, each member of the population comprising a nanoplatelet core including a first semiconductor material and a shell including a second semiconductor material on the surface of the nanoplatelet core, wherein after ligand exchange reaction the population exhibits a quantum yield decrease of less than 50%.

2. The population of semiconductor nanoplatelets according to claim **1,** wherein the ligand is selected from a carboxylic acid, a thiol, an amine, a phosphine, a phosphine oxide an amide, an ester, a pyridine, an imidazole and/or an alcohol.

3. The population of semiconductor nanoplatelets according to claim **1** or claim **2,** wherein the population exhibits fluorescence quantum efficiency decrease of less than 50% after one hour under light illumination.

4. The population of semiconductor nanoplatelets according to anyone of claims **1** to **3,** wherein the population exhibits fluorescence quantum efficiency at 100 °C or above that is at least 80% of the fluorescence quantum efficiency of the population at 20°C.

5. The population of semiconductor nanoplatelets according to claim **4,** wherein the temperature is in a range from 100 °C to 250°C.

6. A process of growth of core/shell nanoplatelets comprising a ME shell on initial colloidal nanoplatelets; said process comprising the steps of injecting the initial colloidal nanoplatelets in a solvent at a temperature ranging from 200°C to 460°C and subsequently a precursor of E or M, wherein said precursor of E or M is injected slowly in order to control the shell growth rate; and wherein the precursor of respectively M or E is injected either in the solvent before injection of the initial colloidal nanoplatelets or in the mixture simultaneously with the precursor of respectively E or M, wherein:
M is selected from group Ib, IIa, IIb, IIIa, IIIb, IVa, IVb, Va, Vb, VIb, VIIb, VIII or mixtures thereof;
E is selected from group Va, VIa, VIIa or mixtures thereof; and
x and y are independently a decimal number from 0 to 5.

7. The process according to claim **6,** comprising successively the steps of:
- heating a solvent at a temperature ranging from 200°C to 460°C;
- injecting in the solvent the initial colloidal nanoplatelets;
- injecting slowly in the mixture the precursor of E and the precursor of M;
- recovering the core/shell nanoplatelets comprising a ME shell on the initial nanoplatelets in the form of nanoplatelets.

8. The process according to claim **6,** comprising successively the steps of:
- heating a solvent at a temperature ranging from 200°C to 460°C;
- injecting a precursor of M in the solvent;
- injecting in the mixture the initial colloidal nanoplatelets;
- injecting slowly in the mixture the precursor of E;
- recovering the core/shell nanoplatelets comprising a ME shell on the initial nanoplatelets in the form of nanoplatelets.

9. The process according to claim **6,** comprising successively the steps of:
- heating a solvent at a temperature ranging from 200°C to 460°C;
- injecting a precursor of E in the solvent;
- injecting in the mixture the initial colloidal nanoplatelets;
- injecting slowly in the mixture the precursor of M;
- recovering the core/shell nanoplatelets comprising a ME shell on the initial nanoplatelets in the form of nanoplatelets.

10. The process according to claim **6,** comprising successively the steps of:
- providing a solvent and a precursor of M;
- heating the mixture of the solvent and the precursor of M at a temperature ranging from 200°C to 460°C;
- injecting in the mixture the initial colloidal nanoplatelets;
- injecting slowly in the mixture the precursor of E;
- recovering in the form of nanoplatelets the core/shell nanoplatelets comprising a ME shell on the initial nanoplatelets.

11. The process according to claim **6,** comprising successively the steps of:
- providing a solvent and a precursor of E;
- heating the mixture of the solvent and the precursor of E at a temperature ranging from 200°C to 460°C;
- injecting in the mixture the initial colloidal nanoplatelets;
- injecting slowly in the mixture the precursor of M;
- recovering in the form of nanoplatelets the core/shell nanoplatelets comprising a ME shell on the initial nanoplatelets.

12. The process according to anyone of claims **6** to **11,** wherein a fraction of the precursor's mixture is mixed with the initial colloidal nanoplatelets before injection in the solvent.

13. The process according to anyone of claims **6** to **12,** further comprising the step of maintaining the mixture at a temperature ranging from 200°C to 460°C during a predetermined duration ranging from 5 to 180 minutes after injection of the second precursor.

14. The process according to anyone of claims **6** to **13** wherein the slow injection of the at least one of the precursor of E or M is performed at a rate ranging from 0.1 to 30 mole/h/mole of M present in the initial nanoplatelets.

15. The process according to anyone of claims **6** to **14,** wherein the initial colloidal nanoplatelets are injected over a period of less than 1 minute.
